# EUROPEAN PATENT APPLICATION

(11) **EP 2 881 043 A1**
(43) Date of publication of application: **10.06.2015**
(21) Application number: 12882120.4
(22) Date of filing: 06.08.2012
(51) Int. Cl.: A61B 8/14, G01N 29/24, A61N 7/00

(54) **ULTRASONIC PROBE HAVING GRADIENT INFORMATION AND DEVICE FOR ULTRASONIC DIAGNOSIS AND TREATMENT USING SAME**

(30) Priority: 30.07.2012 KR 20120083270
(71) Applicant: Nohsn Co., Ltd., Iksan-si, Jeollabuk-do 570-802 (KR); Han, Cheol-Min, Jeollabuk-do 561-803 (KR)
(72) Inventor: HAN, Cheol-Min, Jeonju-si Jeollabuk-do 561-803 (KR)
(74) Representative: Isarpatent
(86) International application number: PCT/KR2012/006217
(87) International publication number: WO 2014/021489

(57) **Abstract**

Disclosed are a probe having gradient information and an ultrasonic diagnosis and treatment apparatus using the same. The probe having gradient information according to the present invention includes a probe unit that generates a ultrasonic signal for diagnosis or treatment and irradiates the generated ultrasonic signal to an object, and a gradient information collecting unit that collects gradient information of the ultrasonic probe in which the probe unit is mounted.

## Description

### [Technical Field]

The present invention relates to an ultrasonic medical apparatus, and more particularly, to an ultrasonic probe having gradient information and an ultrasonic diagnosis and treatment apparatus using the same.

### [Background Art]

Ultrasonic waves are sounds of frequencies above 20 kHz, which cannot be heard by humans, and can create short wavelengths and strong vibration, and thus are not exhibited in normal sounds. Ultrasonic waves are a kind of elastic waves and have a different reflectance at the boundary of different materials respectively, and therefore ultrasonic waves may be reflected or penetrate at an boundary of a medium depending on physical properties of human tissues while being propagated into a human body, and amplitude attenuation may be caused by absorption of the medium. Using these characteristics of ultrasonic waves, an image of the corresponding human tissue may be acquired, and a size and a characteristic of the tissue may be obtained from the acquired image. In addition, when focusing ultrasonic waves, heat may be partially applied to the internal human body, and therefore it is possible to perform treatment of joint pain and destroy and remove a tumor. Thus, ultrasonic waves may be used in diagnosis and treatment.

High intensity focused ultrasound (HIFU) treatment is technology that can necrose a cancer tissue using thermal coagulation and mechanical cavitation by focusing ultrasonic energy on one place (one focal point). Through effect of an excessively high-temperature generated at the focused point, cavitation effect, mechanical effect, and acoustic and chemical effect, coagulative necrosis of a diseased tissue may selectively occur, and proliferation, invasion, and metastasis of a tumor may be prevented.

Meanwhile, in an ultrasonic diagnosis and treatment apparatus, a focal point of ultrasonic waves should be small and accurate for effective treatment. Thus, in order to prevent damage of adjacent tissues of a surgical operation position or a corresponding position rather than the surgical operation position or the corresponding position, or in order to prevent damage of important blood vessels and organs, it is necessary to precisely discern an exact focused position of ultrasonic waves for treatment.

A high intensity focused ultrasound treatment system which is disclosed in Korean Patent Publication No. 10-2011-0074326 may include a pipe-shaped probe support member in which a diagnostic probe for radiating ultrasonic waves for diagnosis may be mounted at an end portion of the probe support member, and therefore it is possible to easily check a treated area and easily monitor a treatment process while performing treatment action of a human body. Nevertheless, an exact focused position of ultrasonic waves for treatment cannot be precisely discerned.

### [Disclosure]

### [Technical Problem]

The present invention is directed to providing an ultrasonic probe having gradient information and an ultrasonic diagnosis and treatment apparatus using the same, which may precisely discern a region for diagnosis and treatment and visualize and schematize a focused position of ultrasonic waves, and therefore it is possible to perform ultrasonic diagnosis and treatment without damaging adjacent tissues rather than a surgical operation position or a corresponding position or damaging important blood vessels and organs.

### [Technical Solution]

One aspect of the present invention provides an ultrasonic diagnosis and treatment apparatus using an ultrasonic probe having gradient information, including: a signal generating unit that generates a transmission signal for diagnosis or treatment for generating a ultrasonic signal for diagnosis or treatment; an ultrasonic probe having gradient information that converts the transmission signal for diagnosis or treatment into the ultrasonic signal for diagnosis or treatment to irradiate the converted ultrasonic signal to an object, converts reflected waves of ultrasonic waves for diagnosis having been reflected on the object and received into an electrical signal to thereby generate a reception signal , and collects gradient information of the ultrasonic probe; a synchronizing unit that synchronizes the transmission signal for treatment or the reception signal with the gradient information; an image generating unit that generates a treatment image using the synchronized reception signal , the synchronized transmission signal for treatment, and gradient information; and a control unit that extracts treatment image information from the treatment image, and generates a control signal for controlling the transmission for treatment signal using the extracted treatment image information.

Here, the ultrasonic diagnosis and treatment apparatus may further include a database unit that stores physical property information of the ultrasonic probe and information about the transmission signal, wherein the control unit may generate the control signal for controlling the transmission signal for treatment using the extracted treatment image information and the information stored in the database unit.

Also, the image generating unit may include a diagnostic image generating unit that generates a diagnostic image using the synchronized reception signal and gradient information, a treatment ultrasonic image generating unit that generates a ultrasonic image for treatment using the information stored in the database unit and the synchronized transmission signal for treatment and gradient information, and a treatment image generating unit that generates the treatment image by mapping the diagnostic image and the ultrasonic image for treatment.

Also, the control unit may control at least one of a type of ultrasonic waves generated from the ultrasonic probe, power of the ultrasonic waves, an ultrasonic beam field, a size of a region, and the number of focused points, by controlling the transmission signal for treatment.

Also, the ultrasonic diagnosis and treatment apparatus may further include a temperature measuring unit that measures a temperature in a region by analyzing an amount of variation in a medium of the object from the reception signal.

Also, the control unit may control at least one of a type of ultrasonic waves generated from the ultrasonic probe, power of the ultrasonic waves, an ultrasonic beam field, a size of a region, and the number of focused points, by controlling transmission signal for treatment using temperature information in the region.

Another aspect of the present invention provides an ultrasonic probe having gradient information including: a probe unit that generates a ultrasonic signal for diagnosis or treatment, irradiates the generated ultrasonic signal to an object, and receives reflected waves returned back from the object; and a gradient information collecting unit that collects gradient information of the ultrasonic probe in which the probe unit is mounted.

### [Advantageous Effects]

According to the present invention, a region for diagnosis and treatment may be precisely discerned and a focused position of ultrasonic waves may be visualized and schematized, and therefore it is possible to perform ultrasonic diagnosis and treatment without damaging adjacent tissues of a surgical operation position or a corresponding position rather than the surgical operation position or the corresponding position, or damaging important blood vessels and organs.

### [Description of Drawings]

FIG. 1 is a configuration diagram illustrating an ultrasonic diagnosis and treatment apparatus using an ultrasonic probe having gradient information according to an embodiment of the present invention;
FIG. 2 is a detailed configuration diagram illustrating an image generating unit of an ultrasonic diagnosis and treatment apparatus according to an embodiment of the present invention;
FIG. 3 is a configuration diagram illustrating an ultrasonic probe having gradient information according to an embodiment of the present invention; and
FIGS. 4 and 5 are diagrams illustrating a beam field and a region according to gradient information of an ultrasonic probe.

### [Modes of the Invention]

Hereinafter, exemplary embodiments of the present invention will be described in detail. However, the present invention is not limited to the exemplary embodiments disclosed below, but can be implemented in various forms. The following exemplary embodiments are described in order to enable those of ordinary skill in the art to embody and practice the invention.

When it is determined that the detailed description of known art related to the present invention may obscure the gist of the present invention, the detailed description thereof will be omitted. The same reference numerals are used to refer to the same element throughout the specification. Terminology described below is defined considering functions in the present invention and may vary according to a user's or operator's intention or usual practice. Thus, the meanings of the terminology should be interpreted based on the overall context of the present specification.

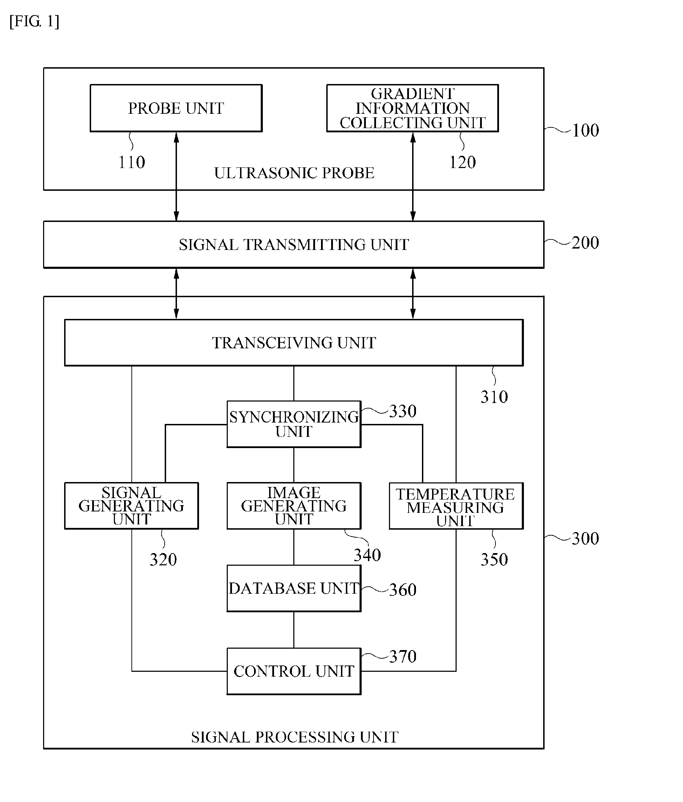

FIG. 1 is a configuration diagram illustrating an ultrasonic diagnosis and treatment apparatus using an ultrasonic probe having gradient information according to an embodiment of the present invention.

As shown in FIG. 1, an ultrasonic diagnosis and treatment apparatus using an ultrasonic probe having gradient information according to an embodiment of the present invention may include an ultrasonic probe 100, a signal transmitting unit 200, and a signal processing unit 300.

The ultrasonic probe 100 having gradient information converts a transmission signal for diagnosis or treatment into a ultrasonic signal for diagnosis or treatment to irradiate the ultrasonic signal to an object, converts reflected waves of ultrasonic waves having been reflected on the object and received into an electrical signal to thereby generate a reception signal , and collects gradient information of the ultrasonic probe. As an example, the ultrasonic probe 100 having gradient information may include a probe unit 110 and a gradient information collecting unit 120. The probe unit 110 receives the transmission signal for diagnosis or treatment generated by the signal processing unit 300, converts the transmission signal into the ultrasonic signal for diagnosis or treatment, irradiates the converted ultrasonic signal to the object, and receives reflected waves returned back from the object. Next, the probe unit 110 converts the received reflected waves into the electrical signal to generate a reception signal , and transmits the reception signal to the signal processing unit 300. The gradient information collecting unit 120 collects gradient information of the ultrasonic probe 100 to transmit the collected gradient information to the signal processing unit 300.

The signal transmitting unit 200 is connected between the ultrasonic probe 100 having gradient information and the signal processing unit 300 to transmit signals between the ultrasonic probe 100 and the signal processing unit 300. As an example, when being provided in a wired manner, the signal transmitting unit 200 may be a wire, a connector, or the like, and when being provided in a wireless manner, the signal transmitting unit 200 may be a transceiver module or the like. In addition, the signal transmitting unit 200 may be implemented within the ultrasonic probe 100, and when the signal processing unit 300 is implemented as a system on chip (SoC) to be implemented within the ultrasonic probe 100, a connector type or transmission standards required in a wireless transmission module may be changed according to an implementation functional unit included in the SoC.

The signal processing unit 300 may include a transceiving unit 310, a signal generating unit 320, a synchronizing unit 330, an image generating unit 340, a temperature measuring unit 350, a database unit 360, and a control unit 370.

The transceiving unit 310 may transmit the transmission signal for diagnosis or treatment generated by the signal generating unit 320 to the ultrasonic probe 100, and receive a reception signal and gradient information of the ultrasonic probe 100 from the ultrasonic probe 100.

The signal generating unit 320 may generate the transmission signal for generating a ultrasonic signal for diagnosis or treatment. In this instance, the transmission signal for treatment includes a focal depth, power of ultrasonic waves, the number of focused points, steering information, and the like.

The synchronizing unit 330 may perform time synchronization between the reception signal and the gradient information of the ultrasonic probe 100. In addition, the synchronizing unit 330 may perform time synchronization between the transmission signal for treatment generated by the signal generating unit 320 and the gradient information of the ultrasonic probe 100. In addition, the synchronizing unit 330 may generate a synchronization signal for generating a treatment image.

The database unit 360 may store physical property information of the ultrasonic probe 100 and information about a transmission signal for generating ultrasonic waves. As an example, the physical property information of the ultrasonic probe 100 may include information about a geometric shape of the ultrasonic probe 100, information about ultrasonic reflection test results of the probe unit 110, and the like. In addition, the information about the transmission signal for generating ultrasonic waves may include a focal depth, a type of characteristics of transmission signal for diagnosis or treatment, frequency and intensity of power, and the like. In addition, the database unit 360 may store ultrasonic beam field for treatment and region information, and the like according to the physical property information of the ultrasonic probe 100 and the information about the transmission signal for generating ultrasonic waves.

The image generating unit 340 may generate the treatment image using the synchronized reception signal, the synchronized transmission signal for treatment, the gradient information of the ultrasonic probe 100, and the information stored in the database unit 360. As an example, the image generating unit 340 may generate a diagnostic image using the synchronized reception signal and the gradient information of the ultrasonic probe 100, and extract beam focusing image information using the synchronized transmission signal for treatment, the gradient information of the ultrasonic probe 100, and the information stored in the database unit 360 to thereby generate a ultrasonic image for treatment. In this instance, the information stored in the database unit 360 may refer to the physical property information of the ultrasonic probe 100, the information about the transmission signal for generating ultrasonic waves, and the like, and the beam focusing image information may include information about a beam field, a size of a region, and the like. In addition, the image generating unit 340 may generate the treatment image by mapping the diagnostic image and the ultrasonic image for treatment according to the synchronization signal of the synchronizing unit 330.

The temperature measuring unit 350 may analyze an amount of variation in a medium of the object by analyzing the reception signal, with respect to the medium of the object which is changed by the ultrasonic signal for treatment. In addition, the temperature measuring unit 350 may measure a temperature in the irradiation region according to the analyzed amount of variation of the medium. As an example, a temperature at which fat is melted is approximately 42 degrees, and therefore the temperature in the irradiation region may be predicted by analyzing a size of an irradiation range or a volume of a melted area and an amount of variation over time.

The control unit 370 may extract treatment image information from the treatment image, and control the signal generating unit 320 using the extracted treatment image information. As an example, the control unit 370 may generate a control signal to control a frequency, amplitude, and the like of the transmission signal generated by the signal generating unit 320, and thereby adjust a type of ultrasonic waves generated from the ultrasonic probe, power of the ultrasonic waves, an ultrasonic beam field, a size of a region, the number of focused points, and the like. In this instance, when controlling the signal generating unit 320, the physical property information of the ultrasonic probe 100 and the information about the transmission signal for generating ultrasonic waves which are stored in the database unit 360 may be used.

In addition, the control unit 370 may control the frequency, amplitude, and the like of the transmission signal using temperature information in the region which is received from the temperature measuring unit 350, and thereby adjust the type of ultrasonic waves generated from the ultrasonic probe, the power of the ultrasonic waves, the ultrasonic beam field, the size of a region, the number of focused points, and the like.

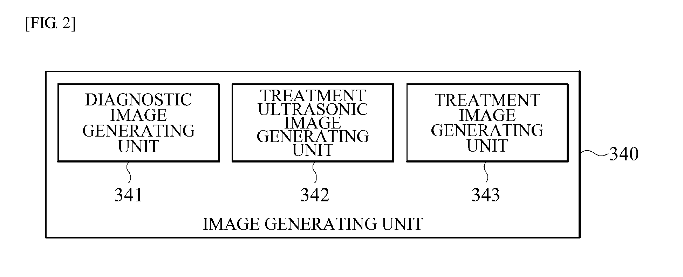

FIG. 2 is a detailed configuration diagram illustrating an image generating unit of an ultrasonic diagnosis and treatment apparatus according to an embodiment of the present invention.

As shown in FIG. 2, the image generating unit 340 may include a diagnostic image generating unit 341, a treatment ultrasonic image generating unit 342, and a treatment image generating unit 343.

The diagnostic image generating unit 341 may generate a diagnostic image using the synchronized reception signal and the gradient information of the ultrasonic probe 100.

The treatment ultrasonic image generating unit 342 may extract beam focusing image information using the synchronized transmission signal for treatment, the gradient information of the ultrasonic probe 100, and the information stored in the database unit 360 to thereby generate the ultrasonic image for treatment. In this instance, the information stored in the database unit 360 may refer to the physical property information of the ultrasonic probe 100, the information about the transmission signal for generating ultrasonic waves, and the like, and the beam focusing image information may include a beam field, a size of a region, and the like.

The treatment image generating unit 343 may generate the treatment image by mapping the diagnostic image and the ultrasonic image for treatment according to the synchronization signal of the synchronizing unit 330.

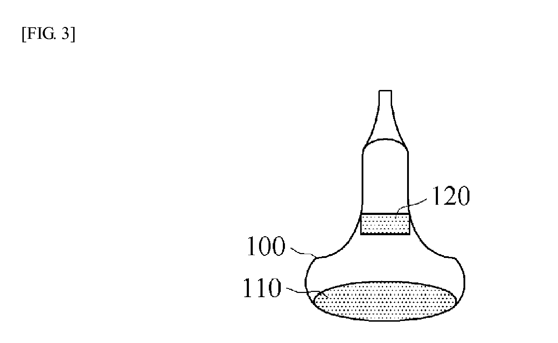

FIG. 3 is a configuration diagram illustrating an ultrasonic probe having gradient information according to an embodiment of the present invention.

As shown in FIG. 3, the ultrasonic probe 100 having gradient information may include the probe unit 110 and the gradient information collecting unit 120. In this instance, in FIG. 3, the appearance of the ultrasonic probe 100 is shown as a convex-shaped appearance, but is not limited thereto. The ultrasonic probe 100 may have an appearance of a variety of shapes such as a linear shape, a phased shape, and the like.

The probe unit 110 may generate a ultrasonic signal for diagnosis or treatment, irradiate the ultrasonic signal to an object, and receive reflected waves returned back from the object. As an example, the probe unit 110 may receive a transmission signal for diagnosis or treatment, convert the received transmission signal into the ultrasonic signal for diagnosis or treatment, irradiate ultrasonic waves to the object, receive reflected waves returned back from the object, and convert the received reflected waves into a reception signal that is an electrical signal. In addition, although not shown, the probe unit 110 may include a transducer that mutually converts the ultrasonic signal and the electrical signal, and the transducer may generally include an aggregation of a plurality of ultrasonic wave vibrators.

The gradient information collecting unit 120 collects gradient information of the ultrasonic probe 100 in which the probe unit 110 is mounted. As an example, the gradient information collecting unit 120 may be a gradient sensor.

In this instance, the probe unit 110 and the gradient information collecting unit 120 may be controlled by a single clock or a synchronization control signal, and thereby may be synchronized in real time so that the gradient information of the ultrasonic probe 100 can be applied to the ultrasonic signal for diagnosis or treatment.

In addition, although not shown, the signal transmitting unit 200 and the signal processing unit 300 of FIG. 1 may be implemented within the ultrasonic probe 100 as a system on chip (SoC).

The ultrasonic probe 100 having gradient information may be used in an ultrasonic diagnosis and treatment apparatus, and the like. When the ultrasonic probe 100 is used in a high intensity focused ultrasound (HIFU) treatment apparatus, the ultrasonic probe 100 may be used in a magnetic resonance (MR) guidance HIFU apparatus, an ultrasonic guidance HIFU apparatus, and the like, but is not limited thereto.

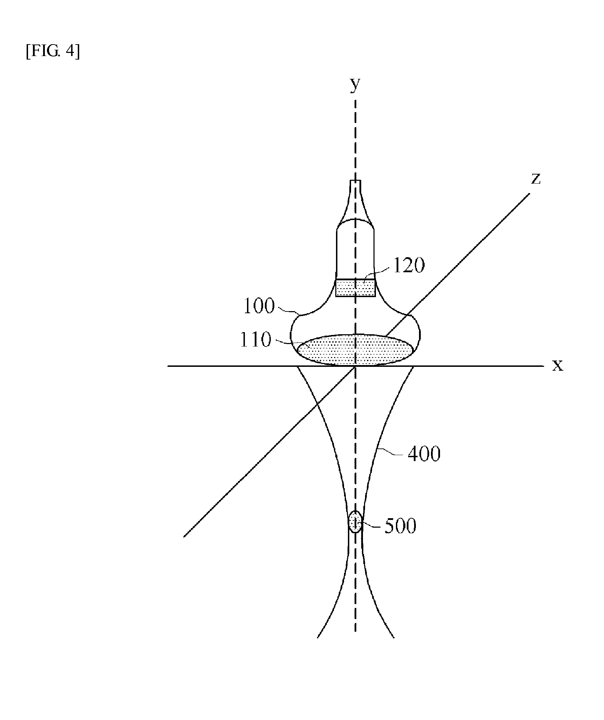

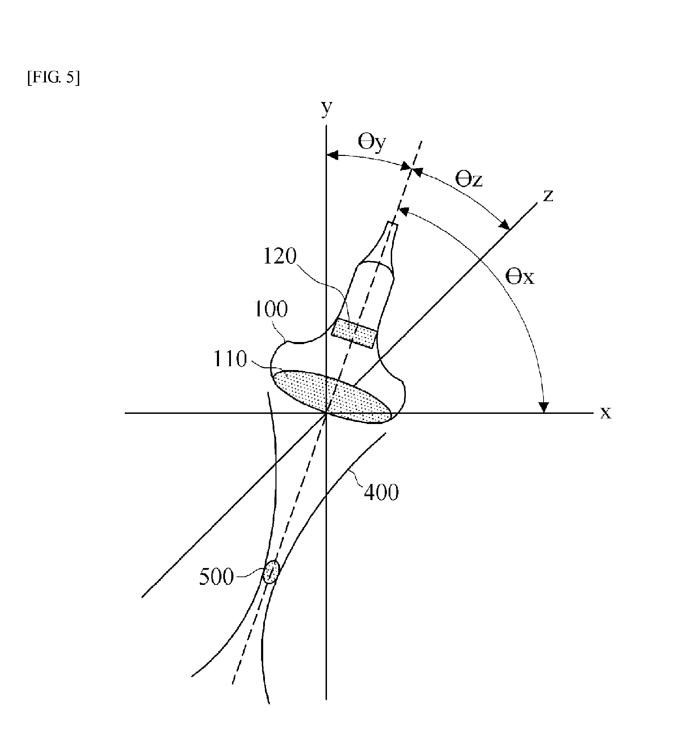

FIGS. 4 and 5 are diagrams illustrating a beam field and a region according to gradient information of the ultrasonic probe 100.

First, when it is assumed that a contact surface of the ultrasonic probe 100 having gradient information and an object is "0" as a reference point, an upper portion of the contact surface may be expressed as "+" and a lower portion thereof may be expressed as "-". However, the present invention is not limited thereto, and a variety of methods in which a coordinate position of a region 500 can be easily expressed may be selected according to the selection of a user or a developer.

Referring to FIG. 4, when a gradient (θₓ, θ_{y}, θ_{z}) of the ultrasonic probe 100 is (0, 0, 0) on an X-Y-Z plane, focal coordinates may be set in a position corresponding to a predetermined focal depth, and a region may be formed in the corresponding position. Next, by mapping information about a beam field 400 and size information of the region 500 corresponding to this position information on a diagnostic image, it is possible to generate a treatment image.

Referring to FIG. 5, when the ultrasonic probe 100 has an arbitrary gradient value (θₓ, θ_{y}, θ_{z}) on an X-Y-Z plane, corresponding focal coordinates may be set depending on (-θₓ, -θ_{y}, -θ_{z}) that is symmetrical to the gradient value and a predetermined focal depth, and the region 500 may be formed in the corresponding position. Next, by mapping information about the beam field 400 corresponding to this position information and size information of the region 500 on the diagnostic image, it is possible to generate the treatment image.

While the invention has been shown and described with reference to certain exemplary embodiments thereof, it will be understood by those skilled in the art that various changes in form and details may be made therein without departing from the spirit and scope of the invention as defined by the appended claims.

## Claims

1. An ultrasonic diagnosis and treatment apparatus using an ultrasonic probe having gradient information, comprising:
a signal generating unit that generates a transmission signal for diagnosis or treatment for generating a ultrasonic signal for diagnosis or treatment;
an ultrasonic probe having gradient information that converts the transmission signal for diagnosis or treatment into the ultrasonic signal for diagnosis or treatment to irradiate the ultrasonic signal to an object, converts reflected waves of ultrasonic waves for diagnosis having been reflected on the object and received into an electrical signal to thereby generate a reception signal, and collects gradient information of the ultrasonic probe;
a synchronizing unit that synchronizes the transmission signal for treatment or the reception signal with the gradient information;
an image generating unit that generates a treatment image using the synchronized reception signal, the synchronized transmission signal for treatment, and gradient information; and
a control unit that extracts treatment image information from the treatment image, and generates a control signal for controlling the transmission signal for treatment using the extracted treatment image information.

2. The ultrasonic diagnosis and treatment apparatus of claim 1, further comprising:
a database unit that stores physical property information of the ultrasonic probe and information about the transmission signal,
wherein the control unit generates the control signal for controlling the transmission signal for treatment using the extracted treatment image information and the information stored in the database unit.

3. The ultrasonic diagnosis and treatment apparatus of claim 2, wherein the image generating unit includes
a diagnostic image generating unit that generates a diagnostic image using the synchronized reception signal and gradient information,
a treatment ultrasonic image generating unit that generates a ultrasonic image for treatment using the information stored in the database unit and the synchronized transmission signal for treatment and gradient information, and
a treatment image generating unit that generates the treatment image by mapping the diagnostic image and the ultrasonic image for treatment.

4. The ultrasonic diagnosis and treatment apparatus of claim 1, wherein the control unit controls at least one of a type of ultrasonic waves generated from the ultrasonic probe, power of the ultrasonic waves, an ultrasonic beam field, a size of a region, and the number of focused points, by controlling the transmission signal for treatment.

5. The ultrasonic diagnosis and treatment apparatus of claim 1, further comprising:
a temperature measuring unit that measures a temperature in a region by analyzing an amount of variation in a medium of the object from the reception signal.

6. The ultrasonic diagnosis and treatment apparatus of claim 5, wherein the control unit controls at least one of a type of ultrasonic waves generated from the ultrasonic probe, power of the ultrasonic waves, an ultrasonic beam field, a size of a region, and the number of focused points, by controlling the transmission signal for treatment using temperature information in the region.

7. An ultrasonic probe having gradient information comprising:
a probe unit that generates a ultrasonic signal for diagnosis or treatment and irradiates the ultrasonic signal to an object; and
a gradient information collecting unit that collects gradient information of the ultrasonic probe in which the probe unit is mounted.
